# EUROPEAN PATENT SPECIFICATION

(11) **EP 3 305 909 B1**
(45) Date of publication and mention of the grant of the patent: **20.07.2022**
(21) Application number: 15893904.1
(22) Date of filing: 23.09.2015
(51) Int. Cl.: C12Q 1/68, C12N 15/11

(54) **SYSTEMIC LUPUS ERYTHEMATOSUS BIOMARKER AND DIAGNOSTIC KIT THEREOF**
BIOMARKER FÜR SYSTEMISCHEN LUPUS ERYTHEMATODES UND DIAGNOSTISCHES KIT DAFÜR
BIOMARQUEUR DU LUPUS ÉRYTHÉMATEUX SYSTÉMIQUE ET KIT DE DIAGNOSTIC ASSOCIÉ

(30) Priority: 03.06.2015 CN 201510297277
(43) Date of publication of application: 11.04.2018
(73) Proprietor: The Second Xiangya Hospital Of Central South University, Changsha, Hunan 410011 (CN); The Regents of the University of Michigan, Ann Arbor, MI 48105 (US)
(72) Inventor: LU, Qianjin, Changsha Hunan 410011 (CN); ZHAO, Ming, Changsha Hunan 410011 (CN); SAWALHA, Amr H., Ann Arbo, MI 48109 (US)
(74) Representative: Keil & Schaafhausen Patentanwälte PartGmbB
(86) International application number: PCT/CN2015/090336
(87) International publication number: WO 2016/192252

(56) References cited:
- WO-A1-2011/028933
- WO-A2-2008/024642
- WO-A2-2013/033627
- WO-A2-2013/101771
- CN-A- 102 140 511
- US-A1- 2013 065 229
- COIT PATRICK ET AL: "Genome-wide DNA methylation study suggests epigenetic accessibility and transcriptional poising of interferon-regulated genes in naive CD4+T cells from lupus patients", JOURNAL OF AUTOIMMUNITY, vol. 43, June 2013 (2013-06), pages 78-84, XP028556151, DOI: 10.1016/j.jaut.2013.04.003
- COIT PATRICK ET AL: "Epigenome profiling reveals significant DNA demethylation of interferon signature genes in lupus neutrophils", JOURNAL OF AUTOIMMUNITY, LONDON, GB, vol. 58, 28 January 2015 (2015-01-28), pages 59-66, XP029204249, ISSN: 0896-8411, DOI: 10.1016/J.JAUT.2015.01.004
- ZHAO MING ET AL: "IFI44L promoter methylation as a blood biomarker for systemic lupus erythematosus", ANNALS OF THE RHEUMATIC DISEASES, vol. 75, no. 11, November 2016 (2016-11), pages 1998-2006, ISSN: 0003-4967(print)

## Description

### FIELD OF THE INVENTION

The present invention relates to a DNA methylation biomarker in peripheral blood from systemic lupus erythematosus, and a diagnostic kit for systemic lupus erythematosus.

### BACKGROUND OF THE INVENTION

SLE (systemic lupus erythematosus, SLE) is a multi-organ, multi-system autoimmune disease characterized by variable clinical manifestations, affecting kidney, neuropsychiatric and blood systems, etc. The early diagnosis of the SLE patients is of great importance in the prevention and treatment of SLE if it can be developed before suffering pivotal organs, thereby improving the quality of life and increasing the survival rate. However, the current biological diagnostic markers are mostly detected after the organ damages occurring in the biochemical and immunological level, therefore, early diagnosis cannot be applied in the organ-involved patients of SLE.

WO 2013033627 discloses a method for diagnosing rheumatoid arthritis or osteoarthritis, determining a rheumatoid arthritis or osteoarthritis prognosis, or determining or predicting a response to treatment for rheumatoid arthritis or osteoarthritis in a subject comprising determining whether at least 2 nucleic acid loci or at least 2 genes in a sample from said subject have methylation states indicative of rheumatoid arthritis, osteoarthritis, a rheumatoid arthritis or osteoarthritis prognosis, or a response to treatment for rheumatoid arthritis or osteoarthritis.

WO 2013101771 A2 discloses a method of treating lupus in a patient, the method comprising administering an anti- interferon type I antibody to a patient.

US 2013065229 A1 discloses a biomarker consisting of between 2 and 35 different nucleic acid probe sets, wherein: (a) a first probe set that selectively hybridizes under high stringency conditions to a nucleic acid selected from the group consisting of HERC6, IFI44L, IFI44, BQ437417, R3HDM2, IFI27, EPSTI1, and LY6E; and (b) a second probe set that selectively hybridizes under high stringency conditions to a nucleic acid selected from the group consisting of HERC6, IFI44L, IFI44, BQ437417, R3HDM2, IFI27, EPSTI1, and LY6E, wherein the first probe set and the second probe set do not selectively hybridize to the same nucleic acid.

Coit Patrick et al: "Genome-wide DNA methylation study suggests epigenetic accessibility and transcription posing of interferon-regulated genes in naïve CD4+T cells from lupus patients" (Journal of Autoimmunity, vl. 43, June 2013, pages 78-84) discloses significant hypomethylation in interferon-regulated genes in naïve CD4+ T cells from lupus patients, including IFIT1, IFIT3, MX1, STAT1, IFI44L, USP18, TRIM 22 and BST2.

Coit Patrick et al: "Epigenome profiling reveals significant DNA demethylation of interferon signature genes in lupus neutrophils" (Journal of Autoimmunity, London, GB, vol. 58, 28 January 2015, pages 59-66) discloses 293 differentially methylated CG sites in neutrophils between lupus patients and control, the majority of which were hypomethylated in lupus neutrophils compared to controls.

### [Technical Problem]

In recent years, the apparent genetic marker research has developed rapidly, many epigenetic markers such as DNA methylation markers, serum microRNA markers were screened and identified these markers for early diagnosis and prognosis of the disease great value. A lot of literatures have identified that hypomethylation of DNA, is involved in the aberrant activation of CD4⁺T cell, thereby, plays a pivotal role in the pathogenesis of SLE. Previous studies identified hypomethylation genes in CD4⁺T cells from SLE patients include CD11a, CD70, CD40L and perforin, etc. Hypomethylation of these genes contributes to their overexpression, thus activating the autoreactive T cells, consequently, leads to the perturbance of SLE. Recently, the phenomenon of methylation in regulatory sequences of CD11a and CD70 promoter has been identified to be used as a secondary diagnosis in SLE. However, this method is limited, for detecting the methylation levels of the two genes can only be done in peripheral blood CD4⁺T cells genome. This requires us to collect more samples of peripheral blood (about 20ml or more), and then using the density gradient centrifugation and MACS methods to isolate the peripheral blood CD4⁺T cells. In addition to large samples and low compliance of the patients, high cost and the time-consuming of the experiments, contribute most to the burden on patients. Additionally, previous methods used to detect the methylation levels of CD11a and CD70 genes, including cloning and sequencing, chip technology, all of which are time-consuming and, without a precise quantitative methylation level, bringing difficulties when applied in clinic.

To date, there is lack of diagnostic criteria for SLE with high sensitivity and specificity, autoantibodies like anti-nuclear antibodies (ANA), exhibiting high sensitivity (95%) but relatively low specificity (65%) in SLE. Due to this, the joint determination of various laboratories indexes was a supplementary diagnostic method in SLE, and resulted in the increasing medical costs and placing a heavy burden on patients. Therefore, developing a new diagnostic marker for SLE will be of great importance and necessary to improve the diagnosis and treatment level of this disease.

### SUMMARY OF THE INVENTION

### [Solution to Problem]

### [Technical Solution]

The object of the present invention is to provide a new use of a DNA methylation biomarker with high sensitivity from the peripheral blood in the early diagnosis of systemic lupus erythematosus patients, and accordingly to provide a use of a diagnostic kit with high sensitivity and specificity for systemic lupus erythematosus, because traditional systemic lupus erythematosus laboratory indexes sensitivity or specificity are not high so as to overcome the deficiencies of the prior art through the present invention.

Long term studies by the inventors have found that the genome-wide epigenetic modifications may play a central role in the development of SLE, especially some aberrant DNA methylations, may be used as early diagnostic markers. It has been confirmed that the methylation levels at two CG sites in the region of IFI44L gene promoter in SLE patients were significantly reduced compared with the healthy controls and the RA disease controls in the SLE patients, when detecting DNA methylation status within 1500bp upstream region of the IFI44L transcriptional start site through large samples of SLE patients, healthy people, and patients with RA. Hence, the methylation levels of two CG sites used for the diagnosis of sensitivity and specificity for SEL are high.

The DNA methylation marker with high sensitivity and specificity in peripheral blood from systemic lupus erythematosus patients is a DNA sequence within 1500bp upstream from a transcription start site of a human IFI44L gene, namely chr1: 79,085,190-79,085,311 (hg19), and a DNA sequence thereof is represented by SEQ ID NO.1.

The DNA sequence contains two CG sites, and the methylation levels thereof in peripheral blood from SLE patients were significantly reduced compared with the healthy controls and also were significantly reduced compared with the RA disease controls.

The present invention also provides a method for detecting the methylation level of the CG site at coordinate 79,085,222 of SEQ ID No.1 sequence in 1500bp upstream region of IFI44L gene transcriptional start site in peripheral blood.

Specifically, the sequencing results analyzed by the software to determine the methylation levels of the DNA sequence within 1500bp upstream from a transcription start site of a subject IFI44L gene in peripheral blood after sequencing by the PCR amplification of target DNA fragment, comprising the following steps: (1) genome-wide DNA extraction in peripheral blood from the subjects; (2) measuring the concentration of the extracted genomic DNA; (3) treating the genomic DNA with bisulfite; (4) amplifying the DNA fragments by the specific PCR primers; (5) examining PCR products by electrophoresis; (6) sequencing the PCR products; (7) analyzing the results from sequencing and obtaining the methylation levels of two CG sites contained in SEQ ID NO. 1.

Another object of the present invention is to provide a use of a diagnostic kit for the diagnosis of SLE, comprising a set of PCR primers as set forth in SEQ ID NO. 2 and SEQ ID NO. 3, and a probe as set forth in SEQ ID No.4, as well as any desired reagents or media, such as genomic DNA extraction from peripheral blood, measuring DNA concentration, bisulfite treatment, PCR analysis, electrophoresis, and pyrosequencing analysis. More specifically, one or more selected components can be involved in the diagnostic kit as follows: deoxyribonucleoside triphosphates, buffers, stabilizers, thermostable DNA polymerase and markers (including fluorescent labels, chemiluminescent labels and radioactive labels).

The methylation levels of SEQ ID NO. 1 sequence at the two CG sites detected by the present invention need to design specific PCR primers amplification of the DNA sequence of SEQ ID NO.1 at the two CG sites. Primer design is based on the target DNA sequence including SEQ ID NO.1 and the DNA sequence including the bases in 200bp nucleotides upstream and downstream sequence, the primer sequences are: upstream primer 5'-TGTGGATAGTGATAATTTGTTATAAAGTAA-3' (as shown in SEQ ID NO.2) ; downstream primer 5'-AACCTCATCCAATCTTAAAACAC-TTATA-3' (as shown in SEQ ID NO. 3), the downstream primer 5'-end is labeled with biotin. The methylation levels of the DNA segment at the two CG sites for pyrosequencing analysis of the present invention needs a special probe, and the primer design is based on a segment of SEQ ID NO.1 in 1500bp upstream region of the IFI44L transcriptional start site as primer sequences: 5'-AATGTTGTTATTTTATTTTAGATAG -3' ((as shown in SEQ ID N0. 4).

### [Advantageous Effects of Invention]

### [Advantageous Effects]

DNA methylation chip (Illumina 450K) was firstly used to screen the differential DNA methylation of genes in peripheral blood cells from SLE patients in the worldwide. The present invention provides SLE early diagnostic kits through large-scale screening of clinical samples using the latest genetic and epigenetic detection technology to find markers for early diagnosis in patients with SLE. In comparison with the samples from healthy group, we found some hypermethylation or hypomethylation of specific genes in the peripheral blood cells from patients with SLE, among which, IFI44L gene showed significant changes in DNA methylation level. By expanding the sample of subjects, further studies have confirmed that the DNA methylation level of the IFI44L promoter in blood from SLE patients was significantly reduced compared with the normal people and RA patients. IFI44L is an IFN-inducible gene located in the type I IFN signaling pathway. IFI44L can be used as a diagnostic maker for SLE as the type I IFN signaling pathway plays an important role in the pathogenesis of SLE.

The present invention overcomes the above noted deficiencies and can be carried out by no more than1ml peripheral blood from SLE patients. The patient's compliance can be largely improved by DNA methylation markers. The diagnostic kit of the present invention detects with high specificity and sensitivity and has followed advantages: less time consuming, simple operation and small amount of sample required for easy on widespread clinical application prospect. The accuracy and specificity of the detecting results (which are over 90%), as well as the efficiency have been greatly improved when using pyrosequencing instrument with specific primers and probes. The development and application of the invention will be of great importance for improving the diagnosis and treatment of patients with SLE, ultimately improving the quality of life and increasing the survival rates.

### BRIEF DESCRIPTION OF THE DRAWINGS

### [Brief Description]

Figure 1 is the specific primer PCR amplified DNA fragment (SEQ ID NO. 1) of electrophoresis.
Figure 2 is the differences of methylation level at CG site 1 in SLE patients, healthy controls and RA patients.
Figure 3 is the differences of methylation level at CG site 2 in SLE patients, healthy controls and RA patients.
Figure 4 is the ROC graph of methylation at level CG site 1 for SLE diagnosis (compared with healthy controls).
Figure 5 is the ROC graph of methylation at level CG site 2 for SLE diagnosis (compared with healthy controls).
Figure 6 is the ROC graph of methylation level at CG site 1 for the differential diagnosis between SLE and RA (compared to RA).
Figure 7 is the ROC graph of methylation level at CG site 2 for the differential diagnosis between SLE and RA (compared with RA).

### DETAILED DESCRIPTION OF THE PREFERRED EMBODIMENTS

### [Embodiments of the present invention]

### Example 1: Preparation of SLE diagnostic kit

The present invention provides a diagnostic kit for SLE consists of: (1) Whole Blood DNA extraction reagents: proteinase K, cell lysate, wash buffer, elution buffer, adsorption column; (2) bisulfite treatment reagents: dilution buffer, conversion buffer, binding buffer, wash buffer, de-sulfonation buffer, elution buffer; (3) PCR Reagents: DNA polymerase, PCR reaction buffer, PCR primers in SEQ ID NO. 2 and SEQ ID NO. 3; (4) electrophoresis reagents of PCR products: the electrophoresis buffer and agarose; (5) pyrosequencing reagents: streptomycin labeled agarose beads, denaturing buffer, sequence primers shown in SEQ ID NO. 4, washing buffer; (6) software for sequencing analysis: PyroMark Q24 Application Software 2.0.

### [Example]

### [Embodiments of the invention]

The following is the detail descriptions of the embodiments of the present invention, including but not limited to, those technological changes according to the invention are protected. Unless otherwise stated, all the technical and scientific terms used herein are generally known to general technical staff in this field.

### Example 1: Preparation of SLE diagnostic kit.

The present invention provides a diagnostic kit for SLE consists of: (1) Whole Blood DNA extraction reagents: proteinase K, cell lysate, wash buffer, elution buffer, adsorption column; (2) bisulfite treatment reagents: dilution buffer, conversion buffer, binding buffer, wash buffer, de-sulfonation buffer, elution buffer; (3) PCR Reagents: DNA polymerase, PCR reaction buffer, PCR primers in SEQ ID NO. 2 and SEQ ID NO. 3; (4) electrophoresis reagents of PCR products: the electrophoresis buffer and agarose; (5) pyrosequencing reagents: streptomycin labeled agarose beads, denaturing buffer, sequence primers shown in SEQ ID NO. 4, washing buffer; (6) software for sequencing analysis: PyroMark Q24 Application Software 2.0.

### Example 2: The application of the diagnostic kit for SLE patients and detection of DNA methylation levels in peripheral blood.

Step 1: SLE patient peripheral blood genomic DNA extraction
(1) Add 0.5ml whole blood to a 1.5ml micro-centrifuge tube, and then add 1 ml of ice cold nuclease free water, mix thoroughly by vortexing or pipetting; (2) Incubate the sample for 5 min at room temperature and Centrifuge for 5 min at 800xg (~3,000 rpm); (3) Discard the supernatant and resuspend the pellet in 150µl of 1xPBS; (4) Add 20µl of Proteinase K Solution, mix by vortexing; (5) Add 350µl of Lysis Solution, mix thoroughly by vortexing or pipetting; (6) Incubate the sample at 56°C for 10 minutes during which the sample and mix by inverting 3 times; (7) Add 180µl of ethanol (96-100%) and mix by pipetting; (8) Transfer the prepared mixture to the spin column. Centrifuge for 1 min at 6,000xg (~8,000 rpm); (9) Place the column into a new collection tube; (10) Add 500µl of Wash Buffer WB I. Centrifuge for 1 min at 8,000xg (~10,000 rpm). Discard the flow-through and place the column back into the collection tube; (11) Add 500µl of Wash Buffer II to the column. Centrifuge for 3 min at maximum speed (≥20,000g, ≥14,000rpm). Discard the collection tube containing the flow-through solution; (12) ≥20000g (≥14000rpm) empty centrifugal I min, the adsorption column was transferred to a new 1.5ml centrifuge tube; (13) Add 80µl of Elution Buffer to the center of the column membrane to elute genomic DNA. Incubate for 2 min at room temperature; (14) Centrifuge for 1 min at 8,000xg (~10,000 rpm); (15) Collect genomic DNA.

Step 2: Determination of the concentration of extracted genomic DNA.

Using the NanoDrop 2000 software from the Thermo Scientific, drawing 1µl DNA extracts to the detecting board and reading the concentration of the sample.

Step 3: Bisulfite treatment of genomic DNA.

(1) According to the DNA concentration and calculate the volume of bisulfite treated DNA (200g); (2) Add 5µl of M-Dilution Buffer to the DNA sample and adjust the total volume to 50µl with water. Mix the sample by flicking or pipetting up and down; (3) Incubate the sample at 37°C for 15 minutes; (4) Prepare the CT Conversion Reagent (CT), minimize its exposure to light: Add 750µl water and 210µl of M-Dilution Buffer to a tube of CT Conversion Reagent, mix at room temperature with frequent vortexing or shaking for 10 minutes; (5) After the above incubation, add 100µl of the prepared CT Conversion Reagent to each sample and mix; (6) Incubate the sample in the dark at 50°C for 12-16 hours; (7) Incubate the sample at 0-4°C (e.g., on ice) for 10 minutes; (8) Add 400µl of M-Binding Buffer to a column and place the column into a provided Collection Tube; (9) Load the sample (from Step 7) into the column containing the M-Binding Buffer. Close the cap and mix by inverting the column several times; (10) Centrifuge at full speed (>10,000xg) for 30 seconds; (11) Add 100µl of M-Wash Buffer to the column. Centrifuge at full speed for 30 seconds. Discard the supernatant; (12) Add 200µl of M-Desulphonation Buffer to the column and let stand at 20-30°C for 18 minutes; (13) Centrifuge at full speed (>10,000xg) for 30 seconds; (14) Add 200µl of M-Wash Buffer to the Wash Buffer. Centrifuge at full speed (>10,000xg) for 30 seconds; (15) Repeat the previous steps once. Discard the supernatant; (16) Centrifuge at full speed (>10,000xg) for 30 seconds. Discard the supernatant; (17) Place the column into a 1.5ml micro-centrifuge tube; (18) Add 10µl of M-Elution Buffer directly to the column matrix. Centrifuge for 30 seconds at full speed (>10,000xg) to elute the DNA; (19) Repeat the previous step once, discard the column. The column collected after the centrifuge tube is the DNA treated with sulfite.

Step 4: Amplifying target DNA fragment and sequencing

These include:
1. Design specific PCR primers to amplify the target DNA fragment followed by sequencing.

Use PyroMark Assay Design 2.0 software to design primers. Input the target DNA sequence (including the bases in 200bp upstream and downstream sequence) into software, and then obtaining specific PCR primers, upstream primer 5'-TGTGGATAGTGATAATTTGTTATAAAGTAA-3' ( as shown in SEQ ID NO. 2), downstream primer 5'-AAC-CTCATCCAATCTTAAAACACTTATA-3' (as shown in SEQ ID NO. 3), which can amplify a PCR product containing the target DNA sequence, with biotin markers at 5 'end of the downstream primer. The sequencing results are average at a length of 355bp, while two CG sites can be detected in the target DNA sequence. The probe sequence is designed as: 5'-AATGTTGTTATTTTATTTTAGATAG-3' (as shown in SEQ ID NO. 4).

DNA fragments by specific PCR primers amplification.

The PCR components are shown in table 1; and the PCR reaction conditions are shown in table 2.

**Table 1: PCR components**

| | |
|---|---|
| 5×buffer | 4µl |
| Upstream primer (10ng/µl) | 0.4µl |
| Downstream primer (10ng/µl) | 0.4µl |
| dNTP | 0.5µl |
| Taq DNA polymerase | 0.5µl |
| Nuclease-Free Water | 12.2µl |
| DNA | 2µl |
| Total volume | 20µl |

**Table 2: Specific PCR conditions of Methylation**

| Step | Temperature | Time | Cycle number |
|---|---|---|---|
| Initial activation | 96°C | 2min | 1 |
| Denaturation | 96°C | 10s | 45 |
| Annealing | 58°C | 30s | |
| Extension | 72°C | 1min | |
| Final extension | 72°C | 10min | 1 |

3. Detect PCR products by agarose gel electrophoresis.

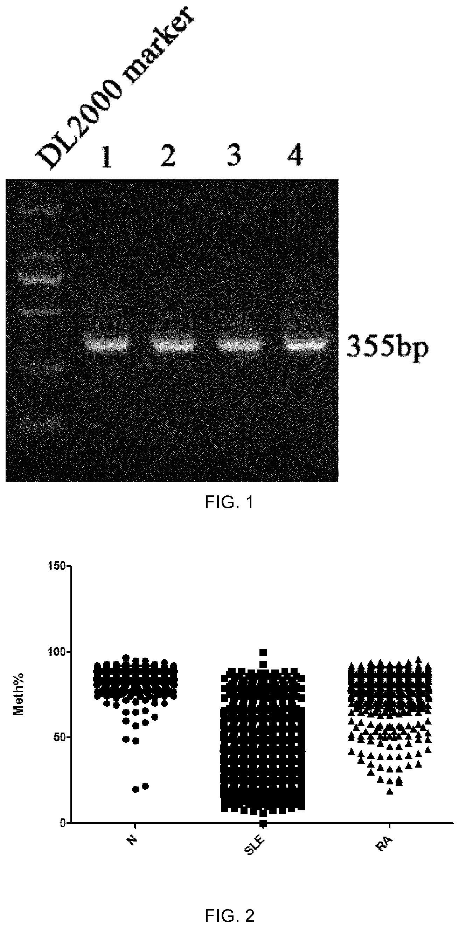

(1) prepare electrophoresis buffer (50xTAE) and dilute to 1×TAE with deionized water; Weigh out the 0.5g agarose and place in an Erlenmeyer flask with 50ml 1×TAE. Dissolve the agarose in a revolving-plate microwave oven. (2) Cool the solution to 50°C, add 2.5µl Ethidium bromide (EB), swirl and mix, then pour the gel onto a taped plate with casting combs in place immediately. Allow more than 60 minutes for solidification. (3) Carefully remove the tape and the gel casting combs, place the gel in a horizontal electrophoresis apparatus. Add 1×TAE electrophoresis buffer to the reservoirs until the buffer just covers the agarose gel. (4) Add 6µl PCR products into each wells and load DNA marks into the first well. (5) Electrophorese the gel at 150-200mA/135V until the required separation has been achieved, usually 25 minutes. (6) After electrophoresis, the gel was placed condensate gel imaging system to observe any obvious specific electrophoretic bands, as shown in figure 1.

Apply Qiagen pyrosequencing Q24 to DNA sequence.

(1) Prepare the reagents and samples; 50ml ethanol (70%) (15ml+35ml), 40ml Denaturation Solution, 10µm sequencing primers, sequencing DNA probe sequence 5'-AATGTTGTTATTTTATT TTAGATAG-3', as shown in SEQ ID NO.4. 50ml wash buffer (45ml H₂O + 5ml Wash Buffer), wash reagent compartment (each with up to thirty times), five high-water. (2) Prepare a mixture called Mix①: total volume is 801/well, including 40ul binding buffer + 2µl streptavidin-coated Sepharose beads + 18µl of high purity water + 20µl PCR product (final plus). (3) Prepare a mixture called Mix②: 10µM × Xµl = 0.3µM ×25µl × n (n is the number of samples required, a Mix② is required every 10 samples addded), calculate X (requiring 10µM sequencing primers), and 25µl × n-X µl is the volum of annealing buffer. (4) Biotinylated PCR products are immobilized on streptavidin coated Sepharose beads. Gently shake the bottle with streptavidin-coated Sepharose beads from side to side until a homogenous solution is obtained. (5) Mix the total amount of streptavidin-coated Sepharose beads (2 µl per sample) and Binding Buffer (40 µl per sample) in a tube. (6) Add high-purity water to a total volume of 80µl per well including the PCR product to be added in step 4. The amount of water depends on the amount of PCR product used. (7) Add the solution prepared in step 2 to a 24-well PCR plate or strips. (8) Add 5-20µl of a well-optimized, biotinylated PCR product to each well of the PCR plate (or strips) according to the plate setup. The total volume per well should be 80µl. (9) Seal the PCR plate to ensure that no leakage is possible between the wells. (10) Agitate the PCR plate (or strips) constantly for at least 5-10 min using a mixer (1400 rpm). Sepharose beads sediment quickly and capturing of beads must take place immediately once the agitation is complete. During immobilization, prepare the vacuum workstation for the sample preparation. (11) Dilute the sequencing primer to 0.3µM in Annealing Buffer, i.e., Mix②. Add 25µl of the solution to each well of a PyroMark Q24 Plate that is to be used. Use one of the supplied PyroMark Q24 Plate Holders as support when preparing and moving the plate.

Procedures of Vacuum Workstation: (1) Ensure that the vacuum Q24 workstation correctly and securely fitted, the base plate 24 preheated (80°C), washing trough, filling the trough (50ml 70% ethanol, 40ml denaturing solution, wash buffer 50ml, 50ml and 70ml high purity water), to open the vacuum pump, the vacuum switch is open, a vacuum is applied in the vacuum apparatus. (2) High water probe, the vacuum is applied, the cleaning process of the probe was filtered off, washed with 70ml high purity water probe, to ensure that the water is transferred to a waste container, the vacuum Jian shut off the vacuum, and placed the rest position (P position). (6) with 70ml high purity water to refill the reagent tank 5. (3) 11.4.8 After the fixed sample and 11.5 ready Pyromark Q24 orifice Mix②, place the PCR plate (or the strips) and PyroMark Q24 Plate on the worktable to ensure consistency with the loading position of the sample plate inch. (4) Switch on the vacuum pump, vacuum applied in the vacuum apparatus. Carefully lower the filter probes into the PCR plate (or strips) to capture the beads containing immobilized template. Hold the filter probes in place for 15 s, carefully remove the vacuum device (agar microspheres precipitate quickly, if the oscillating plate or tube is placed over row Imm, then again one minute before capture oscillation) to ensure that all liquid tank hole and suck out all the microspheres have been captured by filtration probe tip, 70% ethanol reagent tank 5second. (5) The elution buffer 10s. Raise the vacuum means exceed 90°C vertical 5second, to filter probe drainage, vacuum means to hold Pyromark Q24 plate, turn off the vacuum switch (OFF) on the unit by gently rocking vacuum device to release the beads i.e. probes containing the sequencing primer well plates, the vacuum switch closed (OFF), transferred to a vacuum device comprising a reagent of high purity water bath and shaken 10second, the probe is lowered to the second reagent vessel containing pure water and a high vacuum is applied cleaning the probe, with the probe was filtered 70ml high purity water rinsing, vacuum 90°C raising vertical 5 second, drain probe to filter, and then close the vacuum apparatus, placing the rest position, such as a more than one orifice, again filling agent tank, repeating. Turn off the vacuum pump, and the PyroMark Q24 Vacuum Workstation has been assembled correctly and securely.

Using the PyroMark Q24. The concrete step is: (1) Annealing of sequencing primer to samples: Heat the PyroMark Q24 Plate containing the samples at 80°C for 2 min using the PyroMark Q24 Plate Holder (two are supplied with the vacuum workstation) and a heating block. Remove the plate from the plate holder and allow the samples cool to room temperature (15-25°C) for at least 5 min. The plate can now be processed in the PyroMark Q24 Instrument. (2) Preparation of PyroMark Gold Q24 Reagents. Open the PyroMark Gold Q24 Reagents box and remove the vials containing freeze-dried enzyme and substrate mixtures, and the tubes containing nucleotides. Reconstitute the volumes of reagents required according to the handbook supplied with the reagents and fill PyroMark Q24 Cartridge. (3) Starting the run of PyroMark Q24: Open the cartridge gate and insert the filled reagent cartridge, ilnsert the USB stick containing the run file into the USB port at the front of the instrument, using the up and down screen buttons, select "Run" in the main menu and press "OK", select the run file using the up and down screen buttons. To view the contents of a folder, select the folder and press "Select". To go back to the previous view, press "Back". When the run file is selected, press "Select" to start the run. When the run is finished and the instrument confirms that the run file has been saved to the USB memory stick, press "Close". Clean the reagent cartridge.

Step 5: Analysis of methylated DNA sequence level by sequencing results.

According to FIG sequencing results, direct reading software PyroMark value Q24 Application Software 2.0 is given to determine the methylation levels of two CG sites.

### Example 3: To test sensitivity and specificity of the diagnostic kit for SLE

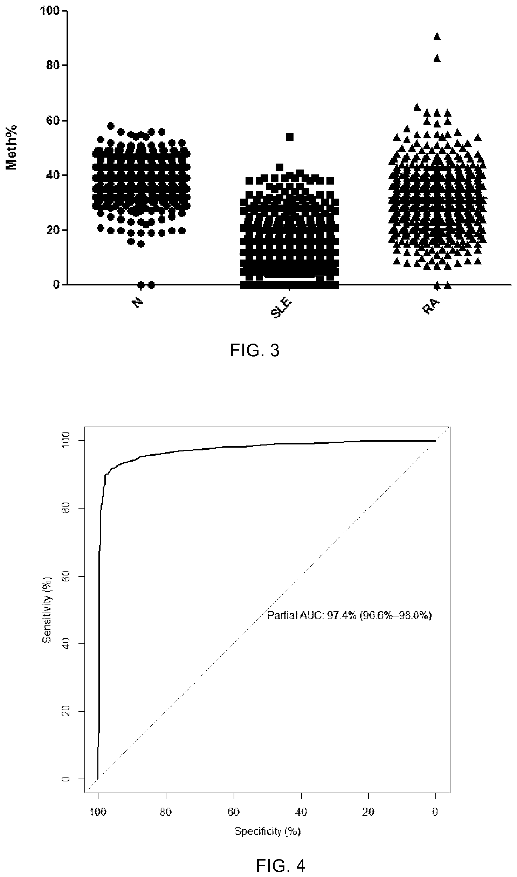

Using the method described in Example 2, detected 1056 cases of SLE patients with 587 healthy controls, 553 cases of rheumatoid arthritis (abbreviated RA) DNA sequences in patients with IFI44L gene transcription start site upstream within -1500bp that methylation level SEQ ID N0.1 contains two CG sites, test results show: the healthy control group, two CG sites showed hypermethylation, two CG sites methylation levels in SLE patients compared with healthy controls and RA patients were significantly lower (as shown in figures 2 and 3).

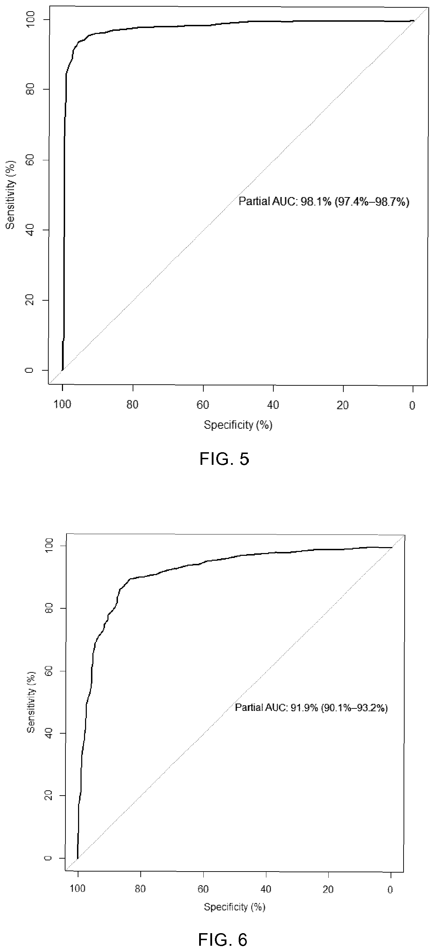

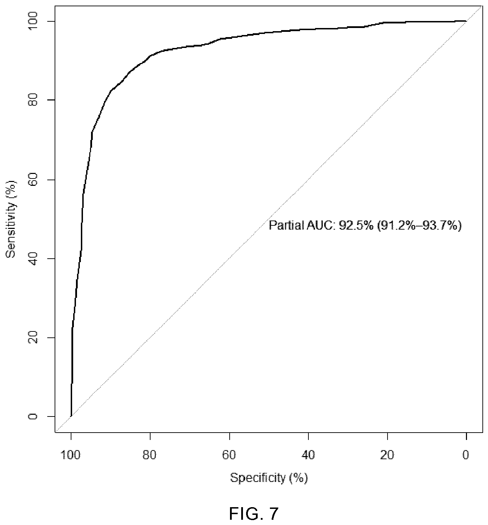

To evaluate the sensitivity and specificity of methylation levels valued by ROC curves in the diagnosis of SLE. The actual area of the Area Under Curve (AUC) is from 0.5 to 1, and it is generally believed that for a diagnostic test, when the area is between 0.5 and 0.7, it is of a low diagnostic value, while the area is between 0.7 to 0.9, the diagnostic value is moderate, or of a high diagnostic value when the area is over 0.9. The analysis of methylation levels at the CG site 1 between SLE patients and healthy controls, with a specificity of 96.10895% and a sensitivity of 91.67513% (as shown in figure 4). The analysis of methylation levels at the CG site 2 between SLE patients and healthy controls, with a specificity of 95.91440% and a sensitivity of 93.50254% (as shown in figure 5). The analysis of methylation levels at the CG site 1 between SLE patients and the RA disease controls, with a specificity of 83.72549% and a sensitivity of 91.67513% (as shown in figure 6). The analysis of methylation levels at the CG site 2 between SLE patients and the RA disease controls, with a specificity of 89.80392% and a sensitivity of 82.53807% (as shown in figure 7).

### [Industrial Applicability]

The present invention provides a use of a diagnostic kit SLE overcomes the deficiencies of the prior art methods of detecting SLE, only need to extract in patients with no more than I ml to find DNA methylation markers from peripheral blood of SLE patients, thus significantly improve patient treatment compliance. High specificity and sensitivity of the kit of the present invention, the inspection took short, simple operation, small amount of sample required for easy on widespread clinical application prospect. Use pyrosequencing instrument with specific primers and probes can greatly reduce DNA methylation inspection time, greatly improve the efficiency and results of laboratory tests to check the accuracy and specificity (can reach more than 90%). The new technology and product development and application will have a great significance for improving the diagnosis and treatment of SLE, the SLE patients to improve quality of life and survival.

## Claims

1. Use of a biomarker from peripheral blood in the early diagnosis of systemic lupus erythematosus, wherein the biomarker is a segment of DNA sequence located in the 1500bp upstream region of the IFI44L human gene transcriptional start site, wherein this the- segment of DNA sequence is located on chr1: 79,085,190-79,085,311 (hg19) as shown in SEQ NO. 1, **characterized in that** the significantly reduced methylation level of the CG site at coordinate 79,085,222 within SEQ NO.1 indicates the presence of systemic lupus erythematosus.

2. A method for detecting the methylation level of the CG site at coordinate 79,085,222 of the SEQ ID No.1 sequence in the 1500bp upstream region of the IFI44L gene transcriptional start site in peripheral blood, comprising the following steps: (1) genome-wide DNA extraction in peripheral blood from the subjects; (2) measuring the concentration of the extracted genomic DNA; (3) treating the genomic DNA with bisulfite; (4) amplifying the DNA fragments represented by SEQ ID NO. 1 by the specific PCR primers; (5) examining the PCR products by electrophoresis; (6) sequencing the PCR products; and (7) analyzing the results from sequencing and obtaining the methylation level of the CG site at coordinate 79,085,222 contained in SEQ ID No. 1.

3. Use of a set of oligonucleotide PCR primers for the detection of the methylation level of the CG site at coordinate 79,085,222 of SEQ ID NO. 1 in the early diagnosis of systemic lupus erythematosus, wherein the set of oligonucleotide PCR primers is complementary to the nucleic acid sequence in the promoter region of IFI44L gene including the nucleic acid sequence in SEQ ID No. 1, wherein the set of nucleotide sequence of PCR primers in upstream primer is set forth in SEQ ID NO. 2 and in downstream primer is set forth in SEQ ID NO. 3.

4. Use of an SLE diagnostic kit for the detection of the methylation level of the CG site at coordinate 79,085,222 of SEQ ID NO. 1 in the early diagnosis of systemic lupus erythematosus, **characterized in that** the kit comprises a set of PCR primers set forth in SEQ ID NO. 2 and SEQ ID NO. 3, wherein the 5' - end of the downstream primer is labeled with biotin.

5. Use of the SLE diagnostic kit for the detection of the methylation level of the CG site at coordinate 79,085,222 of SEQ ID NO. 1 of claim 4, wherein the kit further comprises a probe as defined in SEQ ID NO. 4.

6. Use of a probe for detecting the methylation levels of the nucleic acid sequence at CG site at coordinate 79,085,222 of SEQ ID NO. 1 in the promoter region of IFI44L gene, wherein a set of nucleotide sequence of the probe is set forth in SEQ ID No. 4.

## Patentansprüche

1. Verwendung eines Biomarkers aus peripherem Blut bei der Frühdiagnose von systemischem Lupus erythematodes, wobei der Biomarker ein Segment einer DNA-Sequenz ist, die sich in der 1500 bp stromaufwärts gelegenen Region der Transkriptionsstartstelle des menschlichen Gens IFI44L befindet, wobei sich dieses Segment der DNA-Sequenz auf chr1: 79.085.190 - 79.085.311 (hg19) befindet, wie in SEQ Nr. 1 gezeigt, **dadurch gekennzeichnet, dass** der signifikant reduzierte Methylierungsgrad der CG-Stelle an der Koordinate 79.085.222 innerhalb von SEQ Nr. 1 das Vorhandensein von systemischem Lupus erythematodes anzeigt.

2. Verfahren zum Nachweis des Methylierungsgrads der CG-Stelle an der Koordinate 79.085.222 der Sequenz SEQ ID Nr. 1 in der 1500 bp stromaufwärts gelegenen Region der Transkriptionsstartstelle des Gens IFI44L in peripherem Blut, umfassend die folgenden Schritte: (1) genomweite DNA-Extraktion in peripherem Blut der Probanden; (2) Messen der Konzentration der extrahierten genomischen DNA; (3) Behandeln der genomischen DNA mit Bisulfit; (4) Amplifizieren der durch SEQ ID Nr. 1 dargestellten DNA-Fragmente durch die spezifischen PCR-Primer; (5) Untersuchen der PCR-Produkte durch Elektrophorese; (6) Sequenzieren der PCR-Produkte; und (7) Analysieren der Ergebnisse der Sequenzierung und Ermitteln des Methylierungsgrads der in SEQ ID Nr. 1 enthaltenen CG-Stelle an der Koordinate 79.085.222.

3. Verwendung eines Satzes von Oligonukleotid-PCR-Primern zum Nachweis des Methylierungsgrads der CG-Stelle an der Koordinate 79.085.222 von SEQ ID Nr. 1 bei der Frühdiagnose von systemischem Lupus erythematodes, wobei der Satz von Oligonukleotid-PCR-Primern komplementär zu der Nukleinsäuresequenz in der Promoterregion des Gens IFI44L ist, die die Nukleinsäuresequenz in SEQ ID Nr. 1 beinhaltet, wobei der Satz von Nukleotidsequenzen von PCR-Primern im stromaufwärts gelegenen Primer in SEQ ID Nr. 2 angegeben ist und im stromabwärts gelegenen Primer und im stromabwärts gelegenen Primer in SEQ ID Nr. 3 angegeben ist.

4. Verwendung eines SLE-Diagnosekits zum Nachweis des Methylierungsgrads der CG-Stelle an der Koordinate 79.085.222 von SEQ ID Nr. 1 bei der Frühdiagnose von systemischem Lupus erythematodes, **dadurch gekennzeichnet, dass** das Kit einen Satz von PCR-Primern wie in SEQ ID Nr. 2 und SEQ Nr. 3 angegeben umfasst, wobei das 5'-Ende des stromabwärts gelegenen Primers mit Biotin markiert ist.

5. Verwendung des SLE-Diagnosekits zum Nachweis des Methylierungsgrads der CG-Stelle an der Koordinate 79.085.222 von SEQ ID Nr. 1 nach Anspruch 4, wobei das Kit ferner eine Sonde, wie in SEQ ID Nr. 4 definiert, umfasst.

6. Verwendung einer Sonde zum Nachweis des Methylierungsgrads der Nukleinsäuresequenz an der CG-Stelle an der Koordinate 79.085.222 von SEQ ID Nr. 1 in der Promoterregion des Gens IFI44L, wobei ein Satz von Nukleotidsequenzen der Sonde in SEQ ID Nr. 4 angegeben ist.

## Revendications

1. Utilisation d'un biomarqueur du sang périphérique dans le diagnostic précoce du lupus érythémateux disséminé, dans laquelle le biomarqueur est un segment d'une séquence d'ADN situé dans la région de 1500 pb en amont du site de début de transcription du gène IFI44L, dans laquelle ce segment de séquence d'ADN est situé sur chr1 : 79 085 190-79 085 311 (hg19) tel que présenté dans SEQ ID NO : 1, **caractérisée en ce que** le niveau de méthylation considérablement réduit du site CG au niveau de la coordonnée 79 085 222 à l'intérieur de SEQ ID NO : 1 indique la présence de lupus érythémateux disséminé.

2. Procédé de détection du niveau de méthylation du site CG au niveau de la coordonnée 79 085 222 de : la séquence SEQ ID NO : 1 dans la région de 1500 pb en amont du site de début de transcription du gène IFI44L dans le sang périphérique, comprenant les étapes suivantes : (1) extraction d'ADN à l'échelle du génome dans le sang périphérique des sujets ; (2) mesure de la concentration de l'ADN génomique extrait ; (3) traitement de l'ADN génomique avec du bisulfite ; (4) amplification des fragments d'ADN représentés par SEQ ID NO : 1 par les amorces de PCR spécifiques ; (5) examen des produits de PCR par électrophorèse ; (6) séquençage des produits de PCR ; et (7) analyse des résultats du séquençage et obtention du niveau de méthylation du site CG au niveau de la coordonnée 79 085 222 contenu dans SEQ ID NO : 1.

3. Utilisation d'un jeu d'amorces de PCR oligonucléotidiques pour la détection du niveau de méthylation du site CG au niveau de la coordonnée 79 085 222 de SEQ ID NO : 1 dans le diagnostic précoce du lupus érythémateux disséminé, dans laquelle le jeu d'amorces oligonucléotidiques de PCR est complémentaire de la séquence d'acide nucléique dans la région promotrice du gène IFI44L incluant la séquence d'acide nucléique dans SEQ ID NO : 1, dans laquelle le jeu de séquence nucléotidique d'amorces de PCR dans l'amorce amont est exposé dans SEQ ID NO : 2 et dans l'amorce aval est exposé dans SEQ ID NO : 3.

4. Utilisation d'un kit de diagnostic du SLE pour la détection du niveau de méthylation du site CG au niveau de la coordonnée 79 085 222 de SEQ ID NO : 1 dans le diagnostic précoce du lupus érythémateux disséminé, **caractérisé en ce que** le kit comprend un jeu d'amorces de PCR exposées dans SEQ ID NO : 2 et SEQ ID NO : 3, dans laquelle l'extrémité 5' de l'amorce aval est marquée avec de la biotine.

5. Utilisation d'un kit de diagnostic du SLE pour la détection du niveau de méthylation du site CG au niveau de la coordonnée 79 085 222 de SEQ ID NO : 1 selon la revendication 4, dans laquelle le kit comprend en outre une sonde telle que définie dans SEQ ID NO : 4.

6. Utilisation d'une sonde pour la détection des niveaux de méthylation de la séquence d'acide nucléique au niveau du site CG au niveau de la coordonnée 79 085 222 de SEQ ID NO : 1 dans la région promotrice du gène IFI44L, dans laquelle un jeu de séquences nucléotidiques de la sonde est exposé dans SEQ ID NO : 4 .


### SEQUENCE LISTING

<110> THE SECOND XIANGYA HOSPITAL OF CENTRAL SOUTH UNIVERSITY
<120> SYSTEMIC LUPUS ERYTHEMATOSUS BIOMARKER AND DIAGNOSTIC KIT THEREOF
<130> 106P001895US
<160> 4
<170> Patent In version 3.3
<210> 1
   <211> 122
   <212> DNA
   <213> Homo sapiens
<400> 1
   aatccaatgc tgtcactcca ttccagacag accgaaatac ttggatggcc ctgatgacac 60
   cgtgttctcc tgacttcctt gctttcattt catgtctcag tttaaacttt gatttgctag 120
   ca 122
<210> 2
   <211> 30
   <212> DNA
   <213> Homo sapiens
<400> 2
   tgtggatagt gataatttgt tataaagtaa 30
<210> 3
   <211> 28
   <212> DNA
   <213> Homo sapiens
<400> 3
   aacctcatcc aatcttaaaa cacttata 28
<210> 4
   <211> 25
   <212> DNA
   <213> Homo sapiens
<400> 4
   aatgttgtta ttttatttta gatag 25